# FASCICULE DE BREVET EUROPEEN

(11) **EP 3 347 105 B1**
(45) Date de publication et mention de la délivrance du brevet: **14.08.2019**
(21) Numéro de dépôt: 16763768.5
(22) Date de dépôt: 08.09.2016
(51) Int. Cl.: A61Q 19/04, A61K 8/97, A61K 8/60, A61K 8/9789

(54) **EXTRAIT DE GARDENIA DANS LA COLORATION DE LA PEAU**
GARDENIENEXTRAKT ZUR FÄRBUNG DER HAUT
GARDENIA EXTRACT FOR COLOURING THE SKIN

(30) Priorité: 09.09.2015 FR 1558358
(43) Date de publication de la demande: 18.07.2018
(73) Titulaire: Pierre Fabre Médicament, 92100 Boulogne-Billancourt (FR)
(72) Inventeur: DUPLAN, Hélène, 31320 Auzeville Tolosan (FR); FIORINI-PUYBARET, Christel, 31500 Toulouse (FR); JACQUES-JAMIN, Carine, 31170 Tournefeuille (FR); JOULIA, Philippe, 31290 Villenouvelle (FR); SUBRA, Laurent, 81630 Montgaillard (FR)
(74) Mandataire: Regimbeau
(86) Numéro de dépôt international: PCT/EP2016/071157
(87) Numéro de publication internationale: WO 2017/042257

(56) Documents cités:
- DATABASE GNPD [Online] MINTEL; mars 2013 (2013-03), "Crocetin + cassis 30 Days Supplement", XP002759359, Database accession no. 2038284
- DATABASE GNPD [Online] MINTEL; juillet 2007 (2007-07), "Eye Q Clear essence with DHA, Crocetin & Palm Carotene", XP002759362, Database accession no. 735947
- DATABASE GNPD [Online] MINTEL; octobre 2008 (2008-10), "Crocetin Eye", XP002759363, Database accession no. 1141204
- "Cosmetics for maintaining skin moisture - comprise pptes. obtd. by extracting Gardenia jasminoides or Crocus sativum with water, treating extract and adjusting pH", WPI WORLD PATENT INFORMATION DERWENT, vol. 1, no. 96, 31 octobre 1995 (1995-10-31), XP002021296,
- DATABASE GNPD [Online] MINTEL; janvier 2014 (2014-01), "Bronzoral 1 Solar Sublimating Supplement", XP002759364, Database accession no. 2220161

## Description

La présente invention concerne l'utilisation d'un extrait de Gardénia destinée à colorer la peau.

La couleur de la peau humaine, encore appelée teint, présente une gradation continue du blanc au marron foncé presque noir, avec parfois des tons rosés ou cuivrés. La couleur de la peau est une caractéristique individuelle frappante, souvent considérée comme un marqueur ethnique ou socioéconomique. La quantité et la nature des pigments contenues dans la peau, ainsi que leur répartition, sont les principaux critères déterminant sa couleur. La génétique joue un rôle capital dans la détermination du teint, mais aussi l'exposition au soleil, la plupart des humains bronzant sous l'effet des radiations ultraviolettes. L'absorption de certains médicaments ainsi que des pathologies peuvent provoquer des hyperpigmentations ou des dépigmentations. Trois pigments sont responsables de la couleur de la peau grâce à leur répartition en surface. Le carotène est un pigment dont la couleur varie du jaune à l'orange. Il s'accumule dans la couche cornée et les cellules adipeuses de l'hypoderme et favorise la synthèse de mélanine. Il est présent dans certains végétaux comme la carotte. Le second pigment est l'hémoglobine, lorsque la peau est bien oxygénée, elle donne sa couleur rosée à la peau claire. Une insuffisance d'oxygénation lui donne une couleur bleutée. La mélanine est le pigment le plus important pour la coloration de la peau. Le taux de mélanine détermine sa couleur et assure une plus ou moins grande protection vis-à-vis des rayons ultraviolets. L'épiderme, les cheveux et les poils sont colorés par la mélanine qui se trouve dans la couche la plus profonde de l'épiderme, la couche basale, et est produite par des cellules de grandes tailles, les mélanocytes. La mélanine est synthétisée dans les mélanosomes à partir d'un acide aminé, la tyrosine. La tyrosinase est une enzyme clé dans la synthèse de la mélanine. C'est le taux de mélanosomes et leur taille qui fait varier le taux de mélanine d'une peau à l'autre. La mélanine absorbe les ultraviolets et les infrarouges et capte les radicaux libres qui sont éminemment toxiques et expliquent une grande partie des effets nocifs du soleil.

L'autobronzant est un produit cosmétique utilisé pour obtenir un hâle temporaire similaire à celui obtenu par bronzage, mais sans exposition au soleil. Il doit être appliqué sur le corps pour colorer l'épiderme. L'autobronzant contient du dihydroxyacétone, agent chimique qui permet la coloration de la peau. Il existe donc une demande importante pour développer de nouveaux produits colorant efficacement la peau, faciles à utiliser, par une simple prise orale par exemple.

De façon inattendue, les inventeurs ont trouvé qu'un extrait de Gardénia par voie orale et locale pouvait être utile pour colorer la peau.

Originaire de Chine et du Japon, *Gardenia jasminoides* J. Ellis est un arbuste de la famille des Rubiaceae à feuillage persistant d'environ 1 à 2 m de haut généralement. Cette plante est cultivée dans les pays tropicaux chauds et humides. Les feuilles, d'un vert foncé, sont opposées, elliptiques à ovales-oblongues, de 5 à 10 cm de longueur et de 2 à 7,5 cm de largeur, cunéiformes à la base et aiguës ou acuminées à l'apex avec un pétiole court et à stipules soudées par paires. Les fleurs blanches à ivoire, sont campanulées, de 3 à 4 cm, solitaires, terminales, sessiles et très odorantes. Le fruit est une baie coriace à 5 côtes, de 1 à 1,5 cm de long, ovoïde ou ellipsoïde, surmontée du calice persistant, jaune à rouge à maturité et contenant de nombreuses graines. Le fruit mûr, récolté en automne et séché, est inscrit à la pharmacopée chinoise.

La médecine chinoise prescrit le fruit de Gardénia sous différentes préparations : fruits secs (Zhi-zi), fruits frits (Chaozhi-zi) ou fruits carbonisés (Jiaozhi-zi). En usage interne, il est prescrit comme antipyrétique, contre la dysenterie bacillaire, les infections pulmonaires et urinaires, les hépatites ou comme hémostatique dans les hémorragies nasales provoquées par la fièvre et en usage externe pour traiter les blessures, l'inflammation oculaire, les contusions, les plaies et les furoncles. Dans la médecine japonaise Kampo le fruit est utilisé pour traiter la douleur, les affections pulmonaires et la jaunisse. Ces usages traditionnels peuvent être expliqués par les propriétés pharmacologiques du fruit qui est hémostatique, anti-inflammatoire, stimulant, cholagogue, émétique et diurétique.

Les autres parties du *Gardenia jasminoides* présentent de multiples vertus. Les feuilles, fébrifuges, sont écrasées en Malaisie pour confectionner des cataplasmes pour traiter les migraines, les inflammations pulmonaires. L'écorce, fébrifuge et tonique, est utilisée en cas de fièvre, dysenterie et de douleurs abdominales. En Inde, la racine est utilisée dans la dyspepsie et dans les désordres nerveux. Les fleurs, émollientes, sont utiles pour traiter l'ophtalmie, la blennorragie et la vaginite. Les graines sont utilisées en externe sous forme de pâte pour traiter la jaunisse, le rhumatisme, la diverticulose.

En complément de ces usages médicinaux, les fruits sont également employés pour colorer les denrées alimentaires ou les textiles de couleur jaune à orange en raison de leur richesse en crocines, des pigments identiques à ceux du safran.

Les principaux composés du fruit sont :
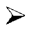 des iridoïdes, représentés majoritairement par le géniposide et gardénoside. Les autres iridoïdes présents ainsi que les composés suivants en quantité plus faible: 6"-O*-trans-*sinapoylgénipin gentiobioside, 6"-O-*trans*-p-coumaroylgenipin gentiobioside, 6"-O-*trans*-cinnamoylgénipin gentiobioside, 6"-O-*trans*-p-coumaroylgéniposide, acide 6'-O-*trans*-p-coumaroylgéniposide, 10-O-succinoylgéniposide, 6'-O-acetylgéniposide, Gardenal-I, Gardenal-II, Gardenal-III, 6-β-hydroxygéniposide,6-α-hydroxygéniposide,6-α-méthoxygéniposide, férétoside, génipin-1-β-gentiobioside, shanzhiside et les acides lamalbidique et picrocrocinique.
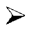 des caroténoïdes tels que l'acide crocéique, la crocétine et les crocines, dérivés glycosylés de la crocétine. On distingue la crocine 1 (crocétine gentiobioside), la crocine 2 (crocétine gentiobioside glucoside) et la crocine 3 (crocétine glucoside),
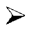 des flavonoïdes : gardénine, quercétine, quercétine-3-rutinoside, quercétine-3-O-glucopyranoside, isoquercitrine, corymbosine, umuhengérine, nicotiflorine.
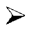 des dérivés caféylquiniques (acide 3-caféoylquinique, acide 4-caféoylquinique), acide 3,4-dicaféoylquinique, acide 3,5-dicaféoylquinique, acide 4,5-dicaféoylquinique, 5-*O-*caféoylquinate d'éthyle, 5-*O*-caféoyl 3-*O*-sinapoylquinate de méthyl, 5-*O*-caféoyl 3-*O*-sinapoylquinate d'éthyle,5-*O*-caféoyl 4-*O*-sinapoylquinate de méthyl, 5-*O*-caféoyl 4-*O-*sinapoylquinate d'éthyle, 3,5-di-*O*-caféoyl-4-*O*-(3-hydroxy-3-méthyl)glutaroylquinate de méthyle, acide 3-O-caféoyl-4-O-sinapoylquinique,3-O-caféoyl-4-0-sinapoylquinate de méthyle, 3-O-caféoyl-5-O-sinapoylquinate de méthyle, acide 3,4-di-O caféoyl-5-O-(3-hydroxy-méthyl) glutaroylquinique, acide 3,5-di-O-caféoyl-4-O-(3-hydroxyméthyl) glutaroylquinique.
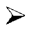 des acides phénols tels que les acides chlorogénique, caféique et 3,4-dihydroxy-benzoique.
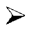 des lignanes : gardénianane A, syringarésinol, pinorésinol, syringarésinol-4-O-β-D-glucopyranoside, laricirésinol, alangilignoside D, lyonirésinol, lyonirésinol-9-O-β-D-glucopyranoside, balanophonine, acide glycosmisique, ficusal et céplignane.
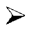 des sucres (mannitol).

La présente invention concerne l'utilisation d'une composition cosmétique destinée à colorer la peau, ladite composition cosmétique comprenant à titre de principe actif un extrait aqueux de *Gardénia.*

D'une façon préférée, l'extrait aqueux de *Gardénia* provient de l'espèce *Gardenia jasminoides.*

Selon un mode de l'invention, l'extrait aqueux de Gardénia utilisé pour colorer la peau, contient une fraction massique de crocines comprise entre 0,1 et 10%, préférentiellement entre 1 et 5%.

La composition selon l'invention peut être formulée pour l'administration à l'être humain. Les compositions selon l'invention peuvent être administrées par voie orale, sublinguale, sous-cutanée, intramusculaire, intraveineuse, transdermique, topique ou bien encore intra-nasale. Avantageusement, la composition selon la présente invention est destinée à une administration par voie topique. D'une façon encore plus avantageuse, la composition selon la présente invention est destinée à une administration par voie orale.

Dans ce dernier cas l'extrait aqueux de Gardénia peut être administré sous formes unitaires d'administration, en mélange avec des supports pharmaceutiques classiques, aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée ou transdermique, topique, intramusculaire, intraveineuse ou intra-nasale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique, la silice ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Dans un mode de réalisation particulier de l'invention, la composition comprend, par dose unitaire, de 10 mg à 1 g d'extrait de Gardénia, préférentiellement de 20 mg à 500 mg, avantageusement de 50 mg à 250 mg et plus préférentiellement de 100 mg à 200 mg ;

Dans un mode particulier de l'invention, la composition comprend, par dose unitaire, de 0,2 mg à 20 mg de crocines, préférentiellement de 1 mg à 5 mg et plus préférentiellement de 2 mg à 4 mg.

Selon un mode de l'invention, l'extrait de Gardénia contient une fraction massique de crocines comprise entre 0,1 et 10%, préférentiellement entre 0,5 et 8%, de manière préférée entre 1 et 5%, encore plus préférentiellement entre 1 et 3%.

Selon un mode de l'invention, l'extrait de Gardénia contient une fraction massique de crocétine comprise entre 0,01 et 5%, préférentiellement entre 0,03 et 3%, encore plus préférentiellement entre 0,1 et 1%.

Selon un mode particulièrement intéressant de l'invention, la composition cosmétique comprend en outre du Paprika et/ou une autre source de caroténoïdes issus de plantes, de microorganismes ou de micro-algues, préférentiellement un extrait de paprika (*Capsicum annuum*), d'*Haematoccus pluvialis* riche en astaxanthine, d'oeillet d'Inde (*Tagetes erecta*) riche en lutéine et zéaxanthine, de tomate source de lycopène, *Dunaliella salina, Xanthophyllomyces dendrorhous* and *Blakeslea trispora* sources de carotènes.

La présente invention concerne également une méthode de traitement cosmétique destinée à colorer la peau, consistant en l'administration d'une composition cosmétique comprenant un extrait aqueux de Gardénia selon l'invention.

La présente invention concerne enfin un procédé d'extraction des fruits de Gardénia.

Les fruits de Gardénia sont extraits par un solvant constitué d'eau ou d'un mélange d'eau et d'un solvant organique, un alcool tel que l'éthanol ou un ester tel que l'acétone. L'extraction peut être réalisée sur des fruits entiers ou broyés grossièrement au préalable. L'ajout d'enzymes, telles que des pectinases permettant d'améliorer l'extraction et/ou leur filtration en fluidifiant les jus d'extraction. L'extraction peut être réalisée par une méthode classique connue de l'homme du métier, en réacteur, par ultrasons ou encore par microondes à une température comprise entre 20°C et 100°C suivant la présence ou non d'enzymes. L'extraction peut être menée à pression atmosphérique ou sous pression avec de l'eau sub-critique.
Après séparation solide-liquide, le filtrat est concentré sur eau, stérilisé puis séché en l'état ou sur un support de séchage, comme la maltodextrine ou la silice. Le séchage est réalisé à des températures inférieures à 80°C afin d'éviter la dégradation des crocines molécules instables à des températures plus élevées. Il peut être réalisé par des techniques connues de l'homme du métier comme par exemple par micro-ondes ou atomisation.

Les exemples suivants illustrent l'invention sans en limiter la portée.

### Exemple 1 : Procédé d'extraction

Les fruits entiers de Gardénia sont extraits à 90°C par l'eau pendant 2 heures. Après séparation solide/liquide, des pectinases sont additionnées. Le filtrat est concentré sur eau *q.s.p.* 40% d'extrait sec, stérilisé puis séché aux micro-ondes.

L'extrait aqueux sec obtenu est une poudre de couleur rouge. Le rendement est de 25% environ. Les teneurs en crocines sont comprises entre 0,1 et 10%, la teneur moyenne en crocines est de l'ordre de 2%.

### Exemple 2 : effets pharmacologiques sur un modèle d'explant de peau humaine

En raison de l'absence de modèle pharmacologique cellulaire et animal, les inventeurs ont mis au point un modèle *ex-vivo* afin de contourner les difficultés liées à l'absorption des actifs. En effet il n'y a pas d'absorption de la crocine sans hydrolyse intestinale au préalable et une faible assimilation de la crocétine et de ces dérivés après passage.

### Protocole

L'objectif de cette étude était d'évaluer l'effet colorant de différents extraits appliqués sur des explants de peau humaine en survie sur 72 heures. Le maintien en survie est effectué dans des plaques de culture 6 puits contenant du milieu de culture. Ces plaques de culture sont placées dans un incubateur à 37°C avec 5% de CO₂. Les études ont été effectuées sur quatre donneurs.

La coloration de chaque milieu contenant des actifs ou témoin est évaluée grâce au Dermoscope C-Cube Edition Clinique de la Société PIXIENCE SAS. Il s'agit de la coloration maximale pouvant être obtenue, soit le niveau 100 % de l'échelle de cartographie de coloration relative à chaque actif. La coloration de chaque explant est mesurée à l'aide du Dermoscope C-Cube Edition Clinique. Au préalable, l'explant est placé dans une solution de milieu sans actif pour rincer l'explant des molécules d'actif présentes en surface de l'explant et qui n'auraient pas pénétré à travers la peau.

### Echantillons testés :

- Témoin : milieu de culture
- oléorésine de Paprika (10% de caroténoïdes)
- extrait aqueux de Gardénia (4% de crocines)
- extrait aqueux de Gardénia hydrolysé par voie chimique (2,7% de crocétine)
- Crocines (100 %)
- Crocétine (87 %), obtenue par hydrolyse chimique des crocines.

Le milieu de culture choisi est un milieu DMEM (Dulbecco's Eagle Modified Medium), supplémenté en glutamine, antifongiques et antibiotique, décrit dans l'étude de Jacques et al. 2010 (Toxicol. In Vitro 24(5), 1426-34).

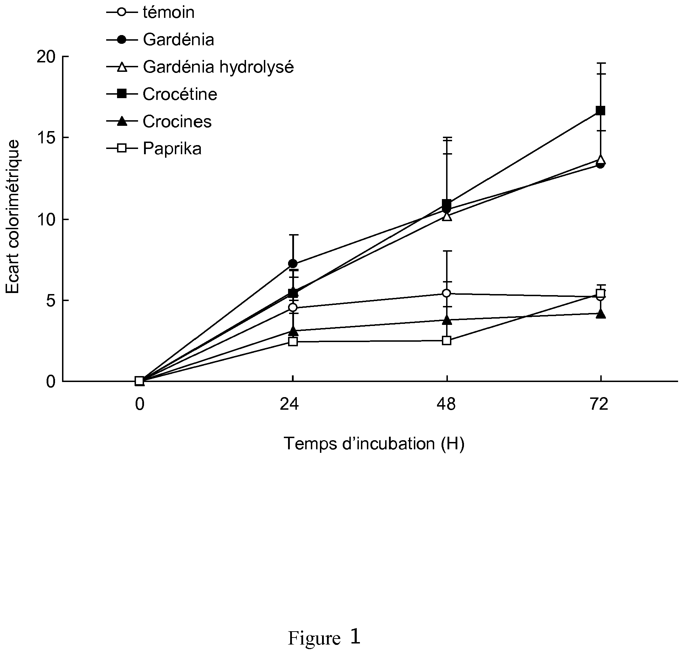

Les photos des explants cutanés ainsi que les écarts colorimétriques, illustrés sur la figure unique annexée, ont permis de montrer le pouvoir colorant important de la Crocétine, du Gardénia et du Gardénia hydrolysé.
Dans ces conditions, le pouvoir colorant du Paprika et des crocines, est inférieur à ceux de la crocétine, du Gardénia et du Gardénia hydrolysé.

Ces études permettent d'apporter la preuve de concept de la coloration des explants liée à l'extrait de Gardénia, mais aussi à l'extrait de Gardénia hydrolysé et de la crocétine. Ces résultats ont été obtenus sur quatre donneurs, à différents temps et avec des expérimentateurs distincts, permettant de valider la reproductibilité de ces résultats.

### Exemple 3 : biodisponibilité des crocines

Peu d'études ont été menées afin d'étudier les propriétés pharmacocinétiques des crocines. L'étude de l'exemple 2 a permis de montrer certes le pouvoir colorant des crocines mais ce résultat est obtenu sur des explants de peau humaine. L'étude réalisée par Xi et al de 2007 (Xi et al., Phytomedicine 14 ; 633-636, 2007) permet de montrer cependant que les crocines ont une biodisponibilité d'au moins 20% chez le rat, ce qui laisse présager des résultats encore supérieurs chez l'homme.

## Revendications

1. Utilisation d'une composition cosmétique destinée à colorer la peau, ladite composition cosmétique comprenant à titre de principe actif un extrait aqueux de *Gardénia.*

2. Utilisation selon la revendication 1 **caractérisée en ce que** ledit extrait provient de l'espèce *Gardenia jasminoides.*

3. Utilisation selon l'une des revendications 1 ou 2 **caractérisée en ce que** ledit extrait contient une fraction massique de crocines comprise entre 0,1 et 10%, préférentiellement entre 1 et 5%.

4. Utilisation selon l'une quelconque des revendications 1 à 3 **caractérisée en ce que** ladite composition est administrée par voie orale.

5. Utilisation selon l'une quelconque des revendications 1 à 4, **caractérisée en ce que** ladite composition comprend en outre du paprika et/ou du β-carotène.

6. Utilisation selon l'une des revendications 1 à 5, **caractérisée en ce que** l'extrait aqueux de Gardénia, est obtenu par :
- une extraction à partir des fruits entiers en présence de pectinases,
- une séparation solide-liquide,
- une stérilisation du filtrat, et
- un séchage à des températures inférieures à 80 °C.

7. Méthode de traitement cosmétique destinée à colorer la peau consistant en l'administration d'une composition cosmétique selon les revendications 1 à 6.

## Patentansprüche

1. Verwendung einer kosmetischen Zusammensetzung, die dazu bestimmt ist, die Haut zu färben, wobei die kosmetische Zusammensetzung als Wirkstoff einen wässrigen Gardenien-Extrakt umfasst.

2. Verwendung nach Anspruch 1, **dadurch gekennzeichnet, dass** der Extrakt von der Gattung *Gardenia jasminoides* stammt.

3. Verwendung nach einem der Ansprüche 1 oder 2, **dadurch gekennzeichnet, dass** der Extrakt einen Massenanteil an Crocinen im Bereich zwischen 0,1 und 10 %, vorzugsweise zwischen 1 und 5 % enthält.

4. Verwendung nach einem der Ansprüche 1 bis 3, **dadurch gekennzeichnet, dass** die Zusammensetzung auf oralem Wege verabreicht wird.

5. Verwendung nach einem der Ansprüche 1 bis 4, **dadurch gekennzeichnet, dass** die Zusammensetzung weiter Paprika und/oder β-Carotin enthält.

6. Verwendung nach einem der Ansprüche 1 bis 5, **dadurch gekennzeichnet, dass** der wässrige Gardenien-Extrakt erhalten wird durch:
- eine Extraktion auf Grundlage der ganzen Früchte in Gegenwart von Pektinasen,
- eine Fest-Flüssig-Trennung,
- eine Sterilisation des Filtrats, und
- ein Trocknen bei Temperaturen unter 80 °C.

7. Kosmetisches Behandlungsverfahren, das dazu bestimmt ist, die Haut zu färben, bestehend in der Verabreichung einer kosmetischen Zusammensetzung nach den Ansprüchen 1 bis 6.

## Claims

1. Use of a cosmetic composition for colouring the skin, said cosmetic composition comprising as active ingredient an aqueous extract of *Gardenia.*

2. Use according to claim 1 **characterized in that** said extract comes from the species *Gardenia jasminoides.*

3. Use according to one of claims 1 or 2 **characterized in that** said extract contains a mass fraction of crocins of 0.1 to 10%, preferentially of 1 to 5%.

4. Use according to any one of claims 1 to 3 **characterized in that** said composition is administered via the oral route.

5. Use according to any one of claims 1 to 4, **characterized in that** said composition further comprises paprika and/or β-carotene.

6. Use according to one of claims 1 to 5, **characterized in that** the aqueous extract of Gardenia is obtained by:
- extraction from whole fruit in the presence of pectinases,
- solid-liquid separation,
- sterilization of the filtrate, and
- drying at temperatures below 80°C.

7. Cosmetic treatment method for colouring the skin consisting in the administration of a cosmetic composition according to claims 1 to 6.
